# EUROPÄISCHE PATENTSCHRIFT

(11) **EP 1 586 926 B1**
(45) Veröffentlichungstag und Bekanntmachung des Hinweises auf die Patenterteilung: **31.12.2008**
(21) Anmeldenummer: 05102819.9
(22) Anmeldetag: 11.04.2005
(51) Int. Cl.: G02B 7/00, A61B 19/00, F16M 11/04

(54) **Stativ für Operationsmikroskope mit einer Gasfeder**
Stand for surgical microscopes having a gas spring
Support pour des microscopes chirurgicaux avec un ressort à gaz

(30) Priorität: 12.04.2004 DE 102004017971
(43) Veröffentlichungstag der Anmeldung: 19.10.2005
(73) Patentinhaber: Leica Microsystems (Schweiz) AG, 9435 Heerbrugg (CH)
(72) Erfinder: Metelski, Andrzej, 8590 Romanshorn (CH)
(74) Vertreter: Rosenich, Paul

(56) Entgegenhaltungen:
- EP-A- 0 433 426
- US-A- 5 609 316
- US-A1- 2002 064 048
- US-A1- 2003 117 703
- US-A1- 2003 178 758
- US-B1- 6 364 268

## Beschreibung

Die Erfindung betrifft ein Stativ für ein Operationsmikroskop, mit einer oder mehreren Gas(druck)fedem.

Um einen möglichst platzsparenden Stativaufbau zu erreichen, verzichten Stative mit Gasfedern auf einen als Gegengewicht ausgestalteten Gegenarm oder eben ein Gegengewicht, das dem Horizontalträger gegenüberliegt und bedienen sich aber dafür der Gasfeder, die die Hebelwirkung des Horizontalträgers, insbesondere unter der Last des Mikroskops, aufnimmt. Hierfür verwendete Gasfedern bestehen aus einem Zylinder, der innen hohl ist und durch einen Kolben in zwei Druckkammern unterteilt ist. Der Kolben ist mit kleinen Löchern (Düsen) versehen, durch die ein Druckausgleich nur verzögert ("gefedert") stattfinden kann. Da der Zylinder ein geschlossenes Drucksystem darstellt, findet der Druckausgleich bis zu gleichen Drücken in beiden Druckkammern statt.

Herkömmliche Stative mit Gasfederabstützung haben sich bewährt, sind jedoch nur in solchen Stativen eingesetzt, die eine mäßige Bewegungshomogenität aufweisen. Für Stative, die über einen größeren Bewegungsraum und/oder komfortablere Bewegungsführung verwendet werden sollen, werden andere Abstützarten verwendet, wie etwa das waagenähnliche Gewicht-Gegengewichts-System.

Herkömmliche Gasfederunterstützungen bei Stativen sind in Abhängigkeit von der zu verwendenden Last austauschbar, d.h., dass für unterschiedliche Lasten unterschiedliche Gasfedern eingesetzt werden. Dieses ist aufgrund der mangelnden Bandbreite des Wirkungsbereichs von herkömmlichen Gasfedern nötig. Es ist erforderlich, die Bandbreite der verschiedenen Gewichte des Operationsmikroskops, z.B. in Abhängigkeit von Zubehörteilen, auf verschieden starke Gasfedern aufzuteilen, damit immer ein ausbalancierter Zustand des Stativs garantiert werden kann. Mit anderen Worten, nähme man z.B. eine Gasfeder mit herkömmlich schmalem Wirkungsbereich und hinge ein Operationsmikroskop in höherer Zubehörausstattung an das Stativ als vorgesehen, balanciere es in eine beliebige Stellung aus und verließe dann diese balancierte Lage, so würde sich der Horizontalträger dann in anderen Lagen selbsttätig bewegen.

Herkömmliche, gasfederabgestützte Stative haben den Nachteil, dass aufgrund der sogenannten "Kosinus-Funktion" der Lasthebelwirkung des Mikroskops entlang seines Bewegungsbogens in der Vertikalen eine unterschiedliche Abstützwirkung in Abhängigkeit von der Winkelstellung des Horizontalträgers zum Vertikalträger vorliegt. In der Schwenkposition des Stativs, in der sich die Last am weitesten entfernt vom Vertikalträger befindet (Horizontalträger und Vertikalträger bilden einen rechten Winkel), ist auch die (Hebel-)Kraft auf die als Stützhebel wirkende Gasfeder am größten.

In der EP-B1-433 426 ist eine Ausgleichsvorrichtung mit einer Gasfeder beschrieben, die ein bogen- oder nlerenförmiges Führungs-Langloch am Vertikalträger umfasst, in der das proximale Ende einer Kolbenstange geführt ist, während der Zylinder als distales Ende der Gasfeder am Horizontalträger schwenkbar befestigt ist. (Im weiteren Verlauf der vorliegenden Anmeldung steht "proximal" für "dem Vertikalträger zugewandt" und "distal" für "dem Vertikalträger abgewandt, zum freien Ende des Horizontalträgers hin".) Dieser Aufbau mit bogenförmigem Führungs-Langloch soll theoretisch verhindern, dass die Hysterese der Gasfeder sich nachteilig bemerkbar macht. Unter Hysterese versteht man allgemein die Abhängigkeit des physikalischen Zustands eines Objekts von vorausgegangenen Zuständen, die auf der Restwirkung (Remanenz) nach Beseitigung der einwirkenden physikalischen Größe bzw. Kraft beruht.

In der Praxis hat sich jedoch gezeigt, dass dieser Aufbau insofern nachteilig ist, als das proximale Ende der Kolbenstange nicht kontinuierlich in dem bogenförmigen Führungs-Langloch wandert, sondern im Anwendungsfall von einer Extremlage zur anderen kniehebelartig springt, was für einen Anwender eine zusätzliche Bewegung über den Springpunkt hinweg erfordert, um die Umstellung der Abstützung in dem bogenförmigen Führungs-Langloch zu erreichen.

Der Erfindung liegt somit die Aufgabe zugrunde, ein verbessertes System mit Gasfeder-Abstützung zu finden, das einerseits auf unterschiedliche Lasten einstellbar ist, um so den Austausch von unterschiedlichen Gasfedern für unterschiedliche Lasten zu vermeiden und andererseits den nachteiligen Kosinus-Effekt des Horizontalträgers unter der Last des Mikroskops zu vermeiden oder so zu vermindern, dass er nicht mehr stört. Außerdem soll der Kniehebelsprungeffekt vermieden werden. Gleichzeitig muss die Gasfeder den typischen Anforderungen für ein Operationsmikroskop-Stativ genügen, d.h. sie muss in der Lage sein, eine Gegenkraft von ca. 2000 N aufzunehmen. So hoch ausgelegte Gasfedern weisen jedoch herkömmlicherweise eine Progression von ca. 18 % auf.

Gelöst wird diese Aufgabe durch die Merkmale des Anspruchs 1, insbesondere durch die Wahl einer Gasfeder mit einer definierten und ausgewählten Progression (herkömmliche Gasfedern haben im Schnitt 11-60 %) von neu unter 10 %, vorzugsweise von unter 9 %, und dieses bei einer vorzugsweise so hohen Kraftaufnahme wie möglich. Vorzugsweise liegt letztere bei ca. 2000 N. Ein möglichst niedriger Progressions-Wert gewährleistet auch eine kleine Hysterese, die gerade in kleinen Bewegungsbereichen, wie sie für eine chirurgische Anwendung typisch sind, störend wirken. Mit anderen Worten, es wird erfindungsgemäß die statische Reibung, die bei kleinen Bewegungen des Horizontalträgers eine wesentliche Rolle spielt, möglichst klein (unter 60 N) gehalten, während die dynamische Reibung, die bei größeren Bewegungen des Horizontalträgers eine Rolle spielt, einen beliebigen, relativ größeren Wert aufweisen kann. Die dynamische Reibung ist nämlich von untergeordneter Bedeutung, weil große Bewegungen des Horizontalträgers nur bei Vorpositionierungen, nicht jedoch bei feinen Manipulationsbewegungen während einer chirurgischen Anwendung erforderlich sind.

Gleichzeitig oder alternativ kann diese Aufgabe dadurch gelöst werden, dass neu anstelle der herkömmlichen Montage der Gasfeder-Kolbenstange am Vertikalträger und des Gasfeder-Zylinders am Horizontalträger der Gasfeder-Zylinder und nicht die Gasfeder-Kolbenstange am verstellbaren Lagerpunkt des Vertikalträgers angelenkt wird. Die Gasfeder-Kolbenstange ist dabei neu vorzugsweise so weit wie möglich am distalen Ende des Horizontalträgers angelenkt. Dadurch wird einerseits die nachteilige Auswirkung der Kosinus-Funktion der Last gemindert, weil das Gewicht des Zylinders vom distalen Ende des Horizontalträgers näher an den Vertikalträger verlagert wird. Außerdem ist dadurch eine geringer störende variable Größe vorhanden, die neu nicht mehr von einer verschieblichen, höheren Zylinder-Masse, sondern von einer verschieblichen, niedrigeren Kolbenstangen-Masse bestimmt ist. Andererseits weist eine möglichst lange Gasfeder (wegen größeren Druckkammern und damit verbundenem niedrigerem möglichen Druck in der Gasfeder) bessere Hysterese-Eigenschaften auf.

Auch wegen größeren Druckkammern (Progressions-Wert ist dann kleiner) und damit verbundenen besseren Hysterese-Eigenschaften ist es bevorzugt, Gasfedern mit möglichst großen Zylinderdurchmessern zu wählen.

Eine weiterhin bevorzugte Ausgestaltung einer speziell für die gewünschten Anwendungszwecke konzipierten Gasfeder weist möglichst kleine Außendurchmesser der Kolbenstange auf. Durch diese konstruktive Maßnahme ist es nochmals möglich, die Hysterese-Eigenschaften zu verbessern und vor allem die statische Reibung, insbesondere die erforderliche "Losbrechkraft" zu Ungunsten einer Erhöhung der dynamischen Reibung zu senken.

Eine weitere, die Gasfeder erfindungsgemäß verbessernde Maßnahme ist die Aufbohrung der Düsen im Kolben. Bei herkömmlichen Gasfedern liegt der Durchmesser dieser Düsen im Zehntel-Millimeter-Bereich, neu jedoch nicht unter 2 mm, vorzugsweise bei 4 mm. Dadurch wird die statische Reibung minimiert.

Des Weiteren ist es bevorzugt, dass der Horizontalträger eine längere Ausladung als bei üblichen Stativen aufweist (900 mm statt 700 mm). Diese Maßnahme bringt bei klein bzw. gleich groß gehaltenen Schwenkwinkeln am Anlenkpunkt der stützenden Gasfeder nicht nur einen größeren Schwenkbereich der Last Operationsmikroskop in der Vertikalen, sondern auch einen größeren Aktionsradius.

Das erfindungsgemäße Stativ weist zusätzlich eine Verstellvorrichtung des Anlenkpunktes der Gasfeder auf. Diese Verstellvorrichtung kann - wie aus dem Stand der Technik bekannt - eine Gewindespindel mit einem Schlitten mit Führung und einem Gelenk sein, die manuell oder motorisch betrieben wird. Es wird ausdrücklich auf eine mögliche Kombinierbarkeit dieser Anmeldung mit einer zeitgleich von der gleichen Anmelderin angemeldeten Erfindung verwiesen. Dort ist eine bidirektional wirkende Verstellvorrichtung offenbart, die auch bei dem hier offenbarten Stativ zum Einsatz gelangen kann.

Üblicherweise ist der Horizontalträger eines Stativs, wie es für Operationsmikroskope verwendet wird, als Parallelogrammträger ausgestaltet. Im Rahmen der Erfindung liegen jedoch auch einfach ausgestaltete Horizontalträger.

Des Weiteren ist die Erfindung nicht auf ein Stativ mit nur einer Gasfeder beschränkt, es sollen auch Stativ-Lösungen mit zwei oder mehreren Gasfedern - insbesondere im Hinblick auf eine Verbesserung der Hysterese-Eigenschaften - im Rahmen der Offenbarung dieser Anmeldung liegen.

Weitere Ausbildungen der Erfindung sind in den Figuren und in den abhängigen Patentansprüchen angegeben.

Die Bezugszeichenliste ist Bestandteil der Offenbarung.

Anhand von Figuren wird die Erfindung symbolisch und beispielhaft näher erläutert.

Die Figuren werden zusammenhängend und übergreifend beschrieben. Gleiche Bezugszeichen bedeuten gleiche Bauteile, Bezugszeichen mit unterschiedlichen Indices geben funktionsgleiche oder ähnliche Bauteile an.

Es zeigen dabei
Fig. 1 - einen Stativaufbau gemäß Stand der Technik;
Fig. 2 - eine erfindungsgemäße Stativanordnung;
Fig. 3a-c - eine schematische Darstellung des sogenannten "Kosinus-Effekts" in drei verschiedenen Lagen und
Fig. 4 - das Federkraftdiagramm einer erfindungsgemäßen Gasfeder.

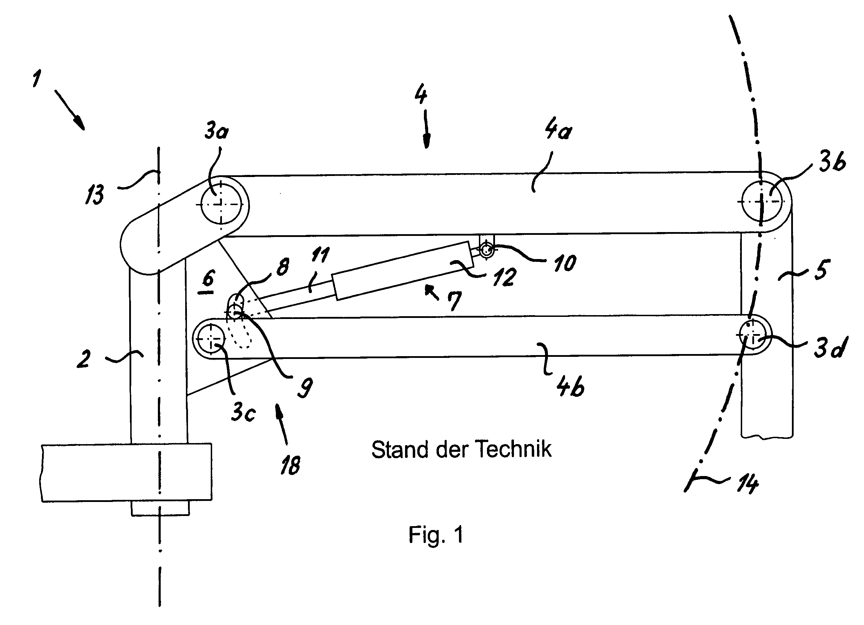

In Fig. 1 ist schematisch eine Stativanordnung 1 gemäß Stand der Technik dargestellt. Das Stativ 1 besteht aus einem Vertikalträger 2 und aus einem Horizontalträger 4, der als Parallelogrammträger mit einem oberen Horizontalträgerarm 4a und einem unteren Horizontalträgerarm 4b ausgebildet ist. An einem Anlenkpunkt 10 an dem oberen Horizontalträgerarm 4a des Horizontalträgers 4 und an einem Anlenkpunkt 9 in einer Platte 6 ist eine Gasfeder 7 angeordnet. Das Stativ 1 weist als Mittel zur positiven Beeinflussung der Hysterese-Eigenschaften eine Verstellvorrichtung 18 auf, die nicht linear wirkt, sondern durch ein bogenförmiges Führungs-Langloch 8 eine radiale Verstellung des Anlenkpunktes 9 ermöglicht.
Die Gasfeder 7 ist mit einem Zylinder 12 am distalen Anlenkpunkt 10 und mit einer Kolbenstange 11 am Anlenkpunkt 9 angeordnet.
Des Weiteren weist diese Stativanordnung Gelenke 3a-d und einen Mikroskopträger 5 auf. Um die Schwenkachse 13 schwenkt der Horizontalträger 4. Dieser wiederum beschreibt bei vertikalen Schwenkbewegungen einen Bewegungsbogen 14.

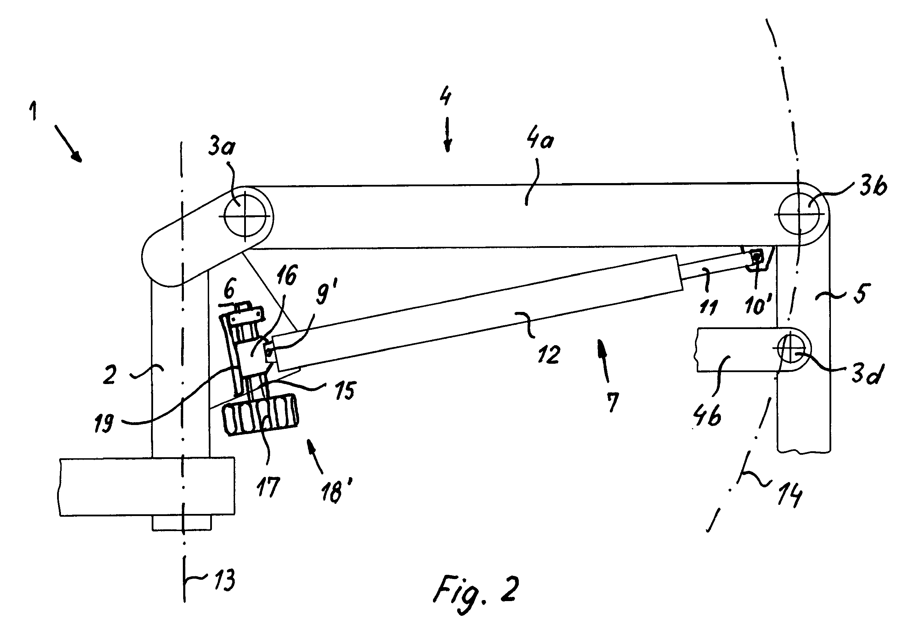

Fig. 2 zeigt eine erfindungsgemäße Stativanordnung 1, die wie die in Fig. 1 dargestellte Ausführung gemäß Stand der Technik einen Vertikalträger 2 und einen als Parallelogrammträger ausgebildeten Horizontalträger 4 aufweist. Des Weiteren weist auch diese Stativanordnung 1 eine Verstellvorrichtung 18' mit einer Gewindespindel 15 auf, die im Vergleich zu der Verstellvorrichtung 18 aus Fig. 1 manuell über Drehungen an einem Verstellrad 17 verstellbar ist und linear wirkt. Wie aus dieser Figur erkennbar, ist die Gasfeder 7 mit der Kolbenstange 11 an einem Anlenkpunkt 10' befestigt, der sich am äußerst möglichen distalen Befestigungspunkt des oberen Horizontalträgerarmes 4a des Horizontalträgers 4 befindet. Des Weiteren ist ersichtlich, dass die Gasfeder 7 nicht nur länger ist, sondern auch einen größeren Zylinderdurchmesser aufweist und nicht mit der Kolbenstange 11, sondern mit dem Zylinder 12 am proximalen Anlenkpunkt 9' befestigt ist.

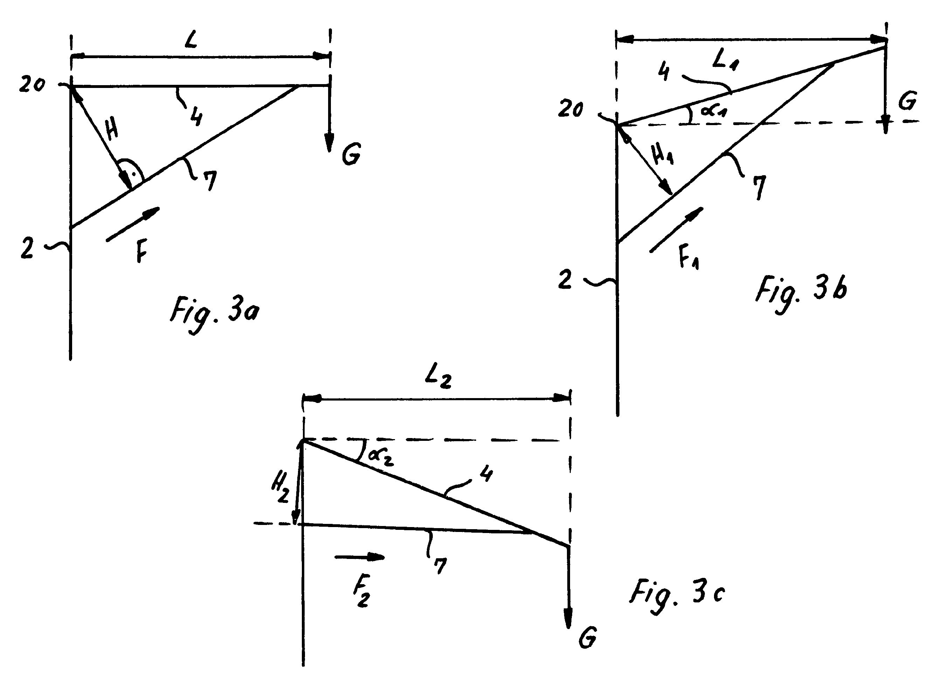

In Fig. 3a-c ist schematisch die Hebelwirkung in Abhängigkeit von verschiedenen Winkeln des Horizontalträgers zum Vertikalträger dargestellt (sogenannter Kosinus-Effekt). Fig. 3a zeigt den Horizontalarm 4 in einer waagerechten Position (Winkel zwischen Vertikalträger 2 und Horizontalarm 4 = 90 Grad). Der Horizontalarm 4 trägt am distalen Ende die Last G und entspricht in dieser Position dem Hebelarm L und die Kraft F, mit der die Gasfeder 7 den Hebelarm L abstützt, befindet sich in einer (virtuellen) Entfernung H vom Anlenkpunkt 20 des Horizontalträgers 4 am Vertikalträger 2. In dieser Position entspricht L-G = H-F. Fig. 3b zeigt den Horizontalarm 4 in einer um einen Winkel α₁ hochgeschwenkten Position. Der Hebelarm L₁ entspricht nun L/cos α₁. Nun ist L₁·G = H₁·F₁. Fig. 3c zeigt den Horizontalarm 4 in einer nach unten um einen Winkel α₂ verschwenkten Position. Der Hebelarm L₂ entspricht nun L/cos α₂ und es gilt die Gleichung L₂-G = H₂-F₂.

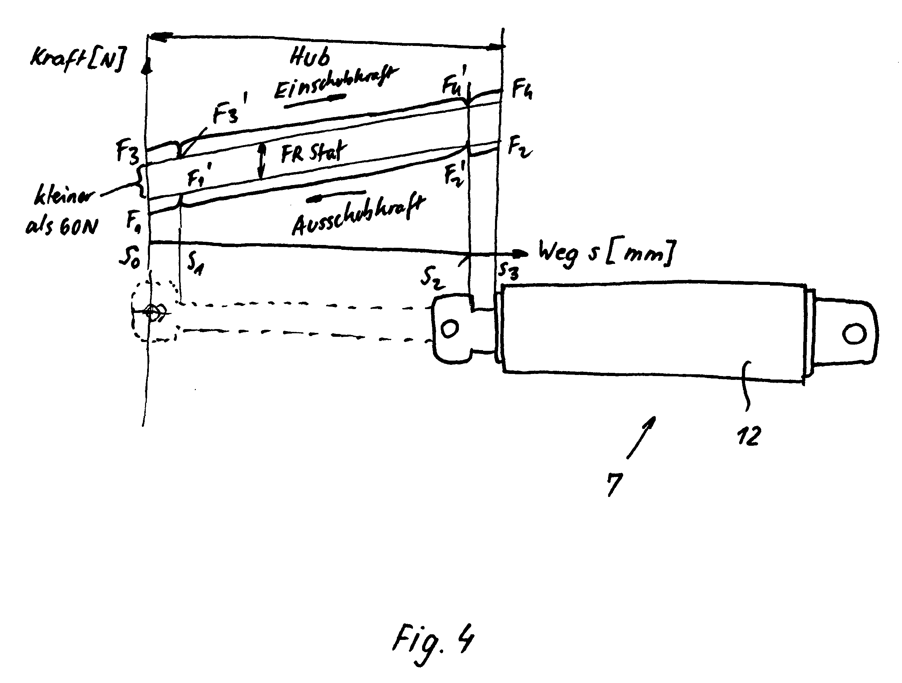

Fig. 4 zeigt das Federkraftdiagramm einer erfindungsgemäßen Gasfeder. Die dynamische Hysterese ist die Differenz F₃-F₁ bzw. F₄-F₂. Die Differenz der statischen Losbrechkraft (statische Hysterese) an einem Wegpunkt s₁ von der Einschubkraft F₃' und der Ausschubkraft F₁' bzw. von F₄'-F₂' beträgt erfindungsgemäß, wie an der Kraftachse ablesbar, weniger als 60 N. Die Parameter der Gasfeder 7 sind erfindungsgemäß so gewählt, dass die Progression unter 10%, vorzugsweise 9% beträgt. Die Progression ist im Federkraftdiagramm als die Steigung von F₁ zu F₂, bzw. F₃ zu F₄ dargestellt.

### Bezugszeichenliste

1 - Stativ
2 - Vertikalträger
3a-d - Gelenk
4 - Horizontalträger
4a - Oberer Horizontalträgerarm
4b - Unterer Horizontalträgerarm
5 - Mikroskopträger
6 - Platte
7- Gasfeder
8 - Bogenförmiges Führungs-Langloch
9 - Proximaler Anlenkpunkt
10 - Distaler Anlenkpunkt
11 - Kolbenstange
12 - Zylinder
13 - Schwenkachse von 4
14 - Bewegungsbogen der Last
15 - Gewindespindel
16 - Schlitten
17 - Handrad
18 - Verstellvorrichtung
19 - Führung von 16
20 - Anlenkpunkt von 4 an 2
α_{1,2} - Winkel zwischen 4 und L_{1,2}
L - Hebelarm
G - Last, Gewicht
F - Kraft
H - Höhe, Entfernung 7 von 20
s₀ - Maximalhub von 11
s_{1,2} - Wegpunkt von 11, an dem gemessen wird
s₃ - Endpunkt
F₁ - Ausschubkraft in s₀
F₂ - Ausschubkraft in s₃
F₃ - Einschubkraft in s₀
F₄ - Einschubkraft in s₃
F₁' - Ausschubkraft in s₁
F₂' - Ausschubkraft in s₂
F₃' - Einschubkraft in s₁
F₄' - Einschubkraft in s₂

## Patentansprüche

1. Stativ (1) für ein Operationsmikroskop, mit einem Vertikalträger (2) und einem daran angelenkten Horizontalträger (4) und einer Gasfeder (7) mit einem Zylinder (12) und einer Kolbenstange (11) beziehungsweise einem Kolben, die an einem proximalen Anlenkpunkt (9) am Vertikalträger (2) und einem distalen Anlenkpunkt (10) am Horizontalträger (4) angelenkt ist, **dadurch gekennzeichnet, dass** der Zylinder (12) der Gasfeder (7) einen Außendurchmesser von 10-100 mm, vorzugsweise 40 mm aufweist, die Kolbenstange (11) der Gasfeder (7) einen Außendurchmesser von 5-50 mm, vorzugsweise 14 mm aufweist und die Gasfeder (7) im inneren des Zylinders (12) einen Kolben mit Bohrungen mit einem Durchmesser von 2-10 mm, vorzugsweise 4 mm aufweist, und dass die Gasfeder (7) eine Progression unter 10%, vorzugsweise unter 9% aufweist, und die statische Reibung des Stativs (1) einen Wert unter 60 N aufweist und-
dass eine Verstellvorrichtung (18') für den proximalen Anlenkpunkt (9) vorgesehen ist. die den Anlenkpunkt (9) höhenverstellbar macht und
dass die Verstellvorrichtung (18') eine Gewindespindel (15), einen Schlitten (16) mit Führung (19) und Anlenkpunkt (9) umfasst und manuell oder motorisch verstellbar ist.

2. Stativ (1) nach einem der vorhergehenden Ansprüche, **dadurch gekennzeichnet, dass** der Horizontalträger (4) als Parallelogrammträger ausgestaltet ist.

3. Stativ (1) nach einem der vorhergehenden Ansprüche, **dadurch gekennzeichnet, dass** die Ausladung des Horizontalträgers (4) innerhalb eines Bereichs von 500-1500 mm liegt, vorzugsweise jedoch 900 mm aufweist.

4. Stativ (1) nach einem der vorhergehenden Ansprüche, **dadurch gekennzeichnet, dass** der distale Anlenkpunkt (10) in der Nähe eines Gelenkes (3b) für die Aufnahme einer Mikroskop-Last (G) am distalen Ende des Horizontalträgers (4) angeordnet ist.

5. Stativ (1) nach einem der vorhergehenden Ansprüche, **dadurch gekennzeichnet, dass** die Gasfeder (7), bestehend aus einem Zylinder (12) und einer Kolbenstange (11), so zwischen Vertikalträger (2) und Horizontalträger (4) angeordnet ist, dass der Zylinder (12) am Vertikalträger (2) und die Kolbenstange (11) am Horizontalträger (4) angelenkt ist.

6. Verwendung einer Gasfeder (7) mit einer Progression von unter 10%, vorzugsweise unter 9 % in einem Stativ für Operationsmikroskope nach einem der vorhergehenden Ansprüche.

## Claims

1. Stand (1) for a surgical microscopy, halving a vertical support (2) and a horizontal support (4) pivoted thereto, and a gas spring (7) having a cylinder (12) and a piston rod (11) and a piston which is pivoted to a proximal pivot point (9) on the vertical support (2) and to a distal pivot point (10) on the horizontal support (4), **characterized in that** the cylinder (12) of the gas spring (7) has an outside diameter of 10-100 mm, preferably 40 mm, the piston rod (11) of the gas spring (7) has an outside diameter of 5-50 mm, preferably 14 mm, and the gas spring (7) in the interior of the cylinder (12) has a piston having bores with a diameter of 2-10 mm, preferably 4 mm, and **in that** the gas spring (7) has a progression of less than 10%, preferably less than 9%, and the static friction of the stand (1) has a value of less than 60 N, **in that** there is provided for the proximal pivot point (9) an adjusting apparatus (18) which allows the height of the pivot point (9) to be adjuster, and **in that** the adjusting apparatus (18) comprises a threaded spindle (15), a carriage (16) with a guide (19) and pivot point (9), and can be adjuster manually or by motor.

2. Stand (1) according to one of the preceding claims, **characterized in that** the horizontal support (4) is configured as a parallelogram support.

3. Stand (1) according to one of the preceding claims, **characterized in that** the projection of the horizontal support (4) lies within a range of 500-1500 mm, but preferably is 900 mm.

4. Stand (1) according to one of the preceding claims, **characterized in that** the distal pivot point (10) is arranged in the vicinity of an articulation (3b) for holding a microscope load (G) at the distal end of the horizontal support (4).

5. Stand (1) according to one of the preceding claims, **characterized in that** the gas spring (7) comprising a cylinder (12) and a piston rod (11) is arranged between the vertical support (2) and horizontal support (4) such that the cylinder (12) is pivoted to the vertical support (2), and the piston rod (11) is pivoted to the horizontal support (4).

6. Use of a gas spring (7) with a progression of less than 10%, preferably less than 9%, in a stand for surgical microscopes according to one of the preceding claims.

## Revendications

1. Pied (1) pour un microscope opératoire, comprenant un support vertical (2) et un support horizontal (4) fixé à celui-ci de manière articulée ainsi qu'un amortisseur pneumatique (7) avec un cylindre (12) et une tige de piston (11) ou un piston qui sont fixés de manière articulée à un point d'articulation proximal (9) sur le support vertical (2) et à un point d'articulation distal (10) sur le support horizontal (4), **caractérisé en ce que** le cylindre (12) de l'amortisseur pneumatique (7) présente un diamètre extérieur de 10 à 100 mm, de préférence de 40 mm, la tige de piston (11) de l'amortisseur pneumatique (7) présente un diamètre extérieur de 5 à 50 mm, de préférence de 14 mm, et l'amortisseur pneumatique (7) présente à l'intérieur du cylindre (12) un piston muni d'orifices ayant un diamètre de 2 à 10 mm, de préférence de 4 mm, et **en ce que** l'amortisseur pneumatique (7) présente une progression intérieure à 10 %, de préférence inférieure à 9 %, et le flottement statique du pied (1) présente une valeur inférieur à 60 N, et **en ce qu'**il est prévu un dispositif de positionnement (18') pour le point d'articulation proximal (9) qui rend le point d'articulation (9) réglable en hauteur, et **en ce que** le dispositif de positionnement (18') comprend une tige filetée (15), un coulisseau (16) avec un guide (19) et un point d'articulation (9) et peut être réglé manuellement ou de manière motorisée.

2. Pied (1) selon l'une des revendications précédentes, **caractérisé en ce que** le support horizontal (4) est réalisé sous la forme d'un support à parallélogramme.

3. Pied (1) selon l'une des revendications précédentes, **caractérisé en ce que** le porte-à-faux du support horizontal (4) est situé dans une plage de 500 à 1500 mm, de préférence cependant égal à 900 mm.

4. Pied (1) selon l'une des revendications précédentes, **caractérisé en ce que** le point d'articulation distal (10) se trouve à l'extrémité distale du support horizontal (4) à proximité d'une articulation (3b) destinée à recevoir une charge de microscope (G).

5. Pied (1) selon l'une des revendications précédentes, **caractérisé en ce que** l'amortisseur pneumatique (7), constitué du cylindre (12) et d'une tige de piston (11), est disposé entre le support vertical (2) et le support horizontal (4) de telle sorte que le cylindre (12) est fixé de manière articulée au support vertical (2) et la tige de piston (11) au support horizontal (4).

6. Utilisation d'un amortisseur pneumatique (7) ayant une Progression inférieure à 10 %, de préférence inférieure à 9 %, dans un pied pour un microscope opératoire selon l'une des revendications précédentes.
